# EUROPEAN PATENT SPECIFICATION

(11) **EP 1 883 388 B1**
(45) Date of publication and mention of the grant of the patent: **09.10.2013**
(21) Application number: 06724584.5
(22) Date of filing: 26.04.2006
(51) Int. Cl.: A61K 8/44, A61Q 15/00

(54) **ACHIEVING A DEODORISING EFFECT WITH AMINOACID DERIVATES**
ERREICHEN EINER DEO-EFFEKT MIT AMINOSÄUREDERIVATE
RÉALISATION D'UN EFFET DÉSODORISANT AVEC DES DERIVES D'ACIDES AMINES

(30) Priority: 21.05.2005 EP 05253150
(43) Date of publication of application: 06.02.2008
(73) Proprietor: Unilever PLC, London EC4Y 0DY (GB); Unilever N.V., 3013 AL Rotterdam (NL)
(72) Inventor: THORNTHWAITE, David, William UNILEVER R & D PORT SUNLIGHT, Merseyside CH63 3JW (GB); TAYLOR, David UNILEVER R & D PORT SUNLIGHT, Bebington, Merseyside CH63 3JW (GB); JAMES, Alexander Gordon UNILEVER R & D PORT SUNLIGHT, Bebington, Merseyside CH63 3JW (GB); STOCKTON, Joanne Elizabeth UNILEVER R & D PORT SUNLIGHT, Bebington, Merseyside CH63 3JW (GB)
(74) Representative: Whaley, Christopher
(86) International application number: PCT/EP2006/003866
(87) International publication number: WO 2006/125503

(56) References cited:
- EP-A- 0 413 528
- WO-A-97/23130
- WO-A-98/12173
- US-A- 3 751 459
- BASRAI M A ET AL: "TOXOCITY OF OXALYSINE AND OXALYSINE-CONTAINING PEPTIDES AGAINST CANDIDA ALBICANS: REGULATION OF PEPTIDE TRANSPORT BY AMINO ACIDS" JOURNAL OF GENERAL MICROBIOLOGY, SOCIETY FOR MICROBIOLOGY, READING, GB, vol. 138, no. PART 11, November 1992 (1992-11), pages 2353-2362, XP001156051 ISSN: 0022-1287

## Description

### Technical Field

The present invention relates to a method of achieving deodorancy on the surface of the human body. The invention involves the use of materials not previously recognised as deodorants.

### Background

There is currently a wide range of deodorant compositions available to the consumer for use on the surface of the human body. However, there are only a limited number of deodorant actives suitable for incorporation in such compositions. Perfumes are suitable deodorant actives; however, these actives typically only mask the malodour produced on the surface of the human body and do not actually reduce the concentration of odiferous molecules produced. Antiperspirant actives are also suitable deodorant actives; however, these materials are typically astringent metal salts and can lead to irritation upon application to the surface of the human body.

There are also a number of organic anti-microbial odour-reducing agents suitable for use as actives in deodorant compositions. These actives tend to be less likely to lead to irritation than antiperspirant actives and yet are more efficacious than simple perfumes. A good description of such actives may be found in "Deodorant Ingredients", S.A.Makin and M.R.Lowry, in "Antiperspirants and Deodorants", Ed. K. Laden (1999, Marcel Dekker, New York).

US 3,751,459 A (Hoffmann-La Roche Inc.) discloses a new antibiotic substance, L-*trans*-2-amino-4-(2-aminoethoxy)-3butenoic acid, a substance named as aminoethoxyvinylglycine [AEVG] in the present specification.

None of the prior art discloses or suggests the compositions of the present invention, nor the use of the particular materials herein described as deodorant actives.

### Summary of the Invention

In a first aspect of the present invention, there is provided a non-therapeutic method of achieving a deodorancy benefit comprising the application of a compound of formula I : R¹R²N-X-O-CH=CH-CH(NH₂)CO₂H or II: R¹R²N-X-O-CH₂CH₂-CH(NH₂)CO₂H where X is an optionally substituted alkylene group comprising two carbons including the possibility that the alkylene group has 3, 4, 5 or more carbon atoms arranged in linear fashion to the surface of the human body.

An objective of the present invention is to provide a highly effective deodorant composition.

A further objective of the present invention is to provide highly effective method of achieving a deodorancy benefit, in particular, a deodorancy benefit upon the surface of the human body.

In the context of this invention, the surface of the human body should be understood to include the hair and skin and to exclude internal surfaces, such as those present in the oral cavity.

### Detailed Description of the Invention

Deodorant compositions according to the present invention are generally applied to the surface of the human body or to articles worn in close proximity thereto. They are particularly effective when applied to the surface of the human body, especially when applied to the more odiferous regions of the human body, such as the underarm regions or feet. They are typically used as cosmetic compositions.

Deodorant compositions according to the present invention comprise a deodorant active of formula I or II:

I R¹R²N-X-O-CH=CH-CH(NH₂)CO₂H

II R¹R²N-X-O-CH₂CH₂-CH(NH₂)CO₂H

where X is an optionally substituted alkylene group comprising two carbons and R¹ and R² are independently H or CH₃.

The requirement that the alkylene group in X comprises two carbon atoms includes the possibility that the alkylene group has 3, 4, 5 or more carbon atoms arranged in linear fashion. Thus, the alkylene group in X may be ethylene, propylene, butylene, or a larger homologue; however, ethylene is the most preferred alkylene group. The alkylene group is optionally substituted, for example with a hydrocarbyl substituent or with a heterohydrocarbyl substituent.

In the context of this invention, the term alkylene should be understood to refer to a saturated linear chain of methylene groups; the term hydrocarbyl should be understood to refer to a substituent having only one or more carbon atoms and one or more hydrogen atoms and the term heterohydrocarbyl should be understood to refer to a substituent having one or more carbon atoms, one or more hydrogen atoms, and one or more hetero-atoms (i.e. one or more atoms that are neither carbon nor hydrogen).

The alkylene group is preferably unsubstituted or substituted with a hydroxymethyl group (-CH₂OH) on the carbon atom bearing the amine group.

With regard to both formula I and formula II, it is preferred that both R¹ and R² are H.

Preferred compounds of formula I or formula II are selected from the following:

H₂N-CH₂-CH₂-O-CH=CH-CH(NH₂)CO₂H

(Aminoethoxyvinylglycine [AEVG])

H₂N-CH₂-CH₂-O-CH₂-CH₂-CH(NH₂)CO₂H

(Aminoethylhomoserine [AEHS])

H₂N-CH(CH₂OH)-CH₂-O-CH=CH-CH(NH₂)CO₂H

(Rhizobitoxine [RhB])

The carbon-carbon double bond in AEVG and RhB is *trains.*

In all chiral compounds of formula I or II, the chirality is preferably L.

Actives having formula I are preferred, particularly those in which the carbon-carbon double bond is *trans.* Preferred actives of formula I are AEVG and RhB, especially AEVG.

The aminoacid of formula I or II is typically employed at a level of from 0.01% to 10% by weight of the total composition. Preferably, the level of incorporation is from 0.05% to 5% and more preferably it is from 0.1% to 2%, by weight of the total composition.

The cosmetic method of achieving a deodorancy benefit described above as the second aspect of the present invention may also include the application of an ester or a salt of a compound of formula I or II to the surface of the human body. In a related aspect of the invention, there is provided a cosmetic method of achieving a deodorancy benefit comprising the application to the surface of the human body of a compound that forms a compound of formula I or II upon the surface of the human body. Such compounds may be esters or salts of compounds of formula I or II *(vide supra)* or they may be amides of compounds of formula I or II, such as acetamidoethoxyvinylglycine [AAEVG], shown below as formula III.

III CH₃CO.NH-CH₂-CH₂-O-CH=CH-CH(NH₂)CO₂H

### Forms of Composition

The compositions of the invention may take any form. Example compositions include wax-based sticks, soap-based sticks, compressed powder sticks, roll-on suspensions or solutions, emulsions, gels, creams, squeeze sprays, pump sprays, and aerosols. Each product form contains its own selection of additional components, some essential and some optional. The types of components typical for each of the above product forms may be incorporated in the corresponding compositions of the invention.

### Carrier material

A carrier material is an essential component of the compositions of the invention. For cosmetic applications, it is essential that the carrier material is cosmetically acceptable. The carrier material may be hydrophobic or hydrophilic, solid or liquid. Preferred carrier materials are liquids. Hydrophobic liquids suitable for use include liquid silicones, that is to say, liquid polyorganosiloxanes. Such materials may be cyclic or linear, examples include Dow Corning silicone fluids 344, 345, 244, 245, 246, 556, and the 200 series; Union Carbide Corporation Silicones 7207 and 7158; and General Electric silicone SF1202. Alternatively, or additionally, non-silicone hydrophobic liquids may be used. Such materials include mineral oils, hydrogenated polyisobutene, polydecene, paraffins, isoparaffins of at least 10 carbon atoms, aliphatic or aromatic ester oils (eg. isopropyl myristate, lauryl myristate, isopropyl palmitate, diisopropyl sebecate, diisopropyl adipate, or C₈ to C₁₈ alkyl benzoates), and polyglycol ethers, for example polyglycol butanol ethers.

Hydrophilic liquid carrier materials, for example water, may also be employed. When water is employed, it is preferred that the pH of the formulation is near to neutral; that is to say, pH 6 to 8. It is also preferred that the pH is non-acidic; that is to say, pH 7 or above. Such pH values give optimum storage stability to the deodorant active and thereby lengthen the useful lifetime of the composition.

Particularly preferred liquid carrier materials are organic solvents. A class of preferred organic solvents are aliphatic alcohols (monohydric or polyhydric, preferably having 2 to 8 carbon atoms) and polyglycol ethers, preferably oligoglycol ethers having only 2 to 5 repeat units. Examples include dipropylene glycol, glycerol propylene glycol, butylene glycol, ethanol, propanol, isopropanol, and industrial methylated spirits. The most preferred organic solvents are aliphatic alcohols, in particular those having 2 to 3 carbon atoms, especially ethanol and isopropanol.

Mixtures of carrier materials may also be used. The total amount of carrier material employed is preferably at least 5%, more preferably from 30% to 99%, and most preferably from 60% to 98% by weight of the composition, excluding any volatile propellant present. Carrier materials should be considered to be all components of the composition, other than deodorant actives of formula I or II.

When organic solvent is present in the composition, it is preferably present at from 30% to 98% by weight of the total weight of the carrier materials; more preferably the organic solvent comprises from 60% to 97% by weight of the carrier materials.

Preferred compositions of the invention comprise a solution of the deodorant active in an organic solvent. Such solutions are preferably homogeneous, preferably having an absorbance, relative to the solvent, of less than 0.2, especially less than 0.1 (for a 1 cm pathlength at 600 nm) measured using a Pharmacia Biotech Ultrospec 200 Spectrophotometer or similar instrument. Suitable organic solvents for use in this embodient include alcohols having from 2 to 3 carbon atoms, especially ethanol and isopropanol. Water may also be present in such compositions.

In a further embodiment of the invention, the deodorant active is suspended in an organic solvent in which it is insoluble. Suitable solvents for use in this embodiment include the aforementioned liquid polyorganosiloxanes.

Suspension of this type can have benefits in terms of the stability of the deodorant active.

In many compositions according to the invention, it is preferred that less than 50%, in particular less than 10%, and especially less than 5% by weight of water is present. Such low levels of water can lead to an enhancement of the performance of the deodorant active of formula I or II, in particular its performance after long term storage of the composition.

Deodorant actives other than those of formula I or II may also be present in compositions according to the invention. Synergies can exist between the deodorant active of formula I or II and such additional deodorants - highly effective odour control being the result.

Additional deodorant actives other than those of formula I or II may be organic anti-microbial agents. Levels of incorporation of such materials are typically from 0.01% to 3%, in particular from 0.03% to 0.5% by weight of the composition, excluding any volatile propellant also present. Most of the classes of agents commonly used in the art can be utilised, for example quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S.A.Makin and M.R.Lowry, in "Antiperspirants and Deodorants", Ed. K. Laden (1999, Marcel Dekker, New York). More preferred additional deodorant actives are polyhexamethylene biguanide (PHMB) salts (eg. PHMB chloride sold as Cosmocil CQ by Arch Chemicals Inc.); 2,4,4'-trichloro,2'-hydroxy-diphenyl ether (triclosan); and 3,7,11-trimethyldodeca-2,6,10-trienol (farnesol).

A particularly preferred additional deodorant active is a transition metal chelator, in particular a material having a high binding constant for iron (III); that is to say, a binding constant for iron (III) of greater than 10¹⁵, preferably greater than 10²⁰, and most preferably greater than 10²⁶, such materials being described in EP 1,248,520 B (Unilever). A particularly preferred material of this class is diethylenetriaminepentaacetic acid (DTPA). Salts of such materials may also be employed. The total amount of transition metal chelator and/or salt thereof is preferably from 0.1% to 5%, more preferably from 0.2% to 3%, and especially from 0.4% to 2% by weight of the composition.

Inorganic anti-microbial agents may also be present as additional deodorant actives. Such materials may also function as anti-perspirant actives. Typically, such materials are astringent metal salts, in particular, aluminium, zirconium and mixed aluminium/zirconium salts, including both inorganic salts, salts with organic anions and complexes. Examples of such astringent salts include aluminium, zirconium and aluminium/zirconium halides and halohydrate salts, such as chlorohydrates. When included, preferred levels of incorporation are from 0.5% to 60%, particularly from 5% to 30% or 40% and especially from 5% or 10% to 30% or 35% by weight of a composition.

Structurants and emulsifiers are further carrier materials that may be employed. Structurants, when employed, are preferably present at from 1% to 30% by weight of a composition, whilst emulsifiers are preferably present at from 0.1% to 10% by weight of a composition. Structurants include cellulosic thickeners such as hydroxy propyl cellulose and hydroxy ethyl cellulose, and dibenzylidene sorbitol. Other structurants include sodium stearate, stearyl alcohol, cetyl alcohol, hydrogenated castor oil, synthetic waxes, paraffin waxes, hydroxystearic acid, dibutyl lauroyl glutamide, alkyl silicone waxes, quaternium-18 bentonite, quaternium-18 hectorite, silica, and propylene carbonate. Emulsifiers include steareth-2, steareth-20, steareth-21, ceteareth-20, glyceryl stearate, cetyl alcohol, cetearyl alcohol, PEG-20 stearate, dimethicone copolyol, and poloxamines.

A perfume is a highly preferred material to include in compositions according to the invention. Suitable perfumes include conventional perfumes, such as perfume oils and also include so-called deo-perfumes, as described in EP 545,556 and other publications. These latter materials may also qualify as additional organic anti-microbial agents. Levels of incorporation are preferably up to 4% by weight, particularly from 0.1% to 2% by weight, and especially from 0.7% to 1.7% by weight of a composition. Synergies can exist between the deodorant active of formula I or II and the perfume - highly effective odour control being the result.

Further emulsifiers desirable in compositions of the invention comprising perfume are perfume solubilisers. Examples include PEG-hydrogenated castor oil, available from BASF in the Cremaphor RH and CO ranges, preferably present at up to 1.5% by weight, more preferably 0.3 to 0.7% by weight.

Other emulsifiers desirable in compositions of the invention are wash-off agents, for example poly(oxyethylene) ethers.

Certain sensory modifiers are further desirable components in the compositions of the invention. Such materials are preferably used at a level of up to 20% by weight of a composition. Emollients, humectants, volatile oils, nonvolatile oils, and particulate solids which impart lubricity are all suitable classes of sensory modifiers. Examples of such materials include cyclomethicone, dimethicone, dimethiconol, isopropyl myristate, isopropyl palmitate, talc, finely divided silica (eg. Aerosil 200), polyethylene (eg. Acumist B18), polysaccharides, corn starch, C12-C15 alcohol benzoate, PPG-3 myristyl ether, octyl dodecanol, C7-C14 isoparaffins, di-isopropyl adipate, isosorbide laurate, PPG-14 butyl ether, glycerol, hydrogenated polyisobutene, polydecene, titanium dioxide, phenyl trimethicone, dioctyl adipate, and hexamethyl disiloxane.

It should be noted that certain components of compositions perform more than one function. Such components are particularly preferred additional ingredients, their use often saving both money and formulation space. Examples of such components include ethanol, isopropyl myristate, and silica.

Further additional components that may also be included are colourants and preservatives, for example C₁-C₃ alkyl parabens.

Aerosol compositions according to the invention generally comprise a volatile propellant. The level of incorporation of the volatile propellant is typically from 30 to 99 parts by weight and particularly from 50 to 95 parts by weight. Non-chlorinated volatile propellant are preferred, in particular liquefied hydrocarbons or halogenated hydrocarbon gases (particularly fluorinated hydrocarbons such as 1,1-difluoroethane and/or 1-trifluoro-2-fluoroethane) that have a boiling point of below 10°C and especially those with a boiling point below 0°C. It is especially preferred to employ liquefied hydrocarbon gases, and especially C₃ to C₆ hydrocarbons, including propane, isopropane, butane, isobutane, pentane and isopentane and mixtures of two or more thereof. Preferred propellants are isobutane, isobutane/isopropane, isobutane/propane and mixtures of isopropane, isobutane and butane.

Other propellants that can be contemplated include alkyl ethers, such as dimethyl ether or compressed non-reactive gases such as air, nitrogen or carbon dioxide.

A particularly preferred aerosol compositions according to the invention has a carrier material comprising ethanol, water, and dimethyl ether, such a carrier system being highly compatible with the deodorant active according to formula I and II and generally enabling the formulation of a single phase composition.

### Methods of Manufacture

Compositions according to the invention may be prepared by suspending or dissolving an aminoacid compound of formula I or II in a carrier material, preferably with sufficient agitation to achieve a homogeneous mixture. When the aminoacid compound is suspended in the carrier material, it is preferred that the aminoacid compound is first ground to a mean particle size of less 100 microns.

### Examples

The deodorancy performance of a most preferred deodorant active for use in accordance with the present invention was assessed in the following tests. All percentages indicated are by weight, unless otherwise indicated.

In a first test, the test product was a 0.25% aqueous AEVG solution and the control product was water. The products were dosed at 400 mg per axilla using a conventional pump spray dispenser. The protocol indicated below was followed. The results are shown in Table 1.

### Product Application and Deodorancy Protocol

The panel employed comprised 50 individuals who had been instructed to use control ethanolic deodorant products during the week prior to the test. At the start of the test, panellists were washed with unfragranced soap and the different products applied to each axilla. (Product application was randomised to take into account any left/right bias). Panellists were instructed not to consume spicy food or alcohol, and not to wash under their own axillae, during the duration of the test. At least three expert assessors determined the intensity of axillary odour 5 hours and 24 hours after application, scoring the malodour intensity on a scale of 0-5. After each 24 hour assessment, the panellists were re-washed, and products re-applied, as above. The procedure was repeated 4 times. At the end of the test the data were analysed using standard statistical techniques.

In a second test, the test product was 0.25% AEVG in aqueous ethanol (20:80 w/w) and the control product was aqueous ethanol (20:80 w/w). The protocol employed was the same as that used for the first test. The results from this test are also shown in Table 1.

**Table 1**

| **Test product** | **Malodour score after...** | |
|---|---|---|
| | **5 hours** | **24 hours** |
| Test 1 | | |
| AEVG in water | 1.98 | 2.17 |
| Water control | 2.37 | 2.64 |
| Test 2 | | |
| AEVG in aqueous ethanol | 1.63 | 1.86 |
| Aqueous ethanol control | 1.81 | 2.09 |

The differences between the malodour scores for the test product and the relevant control were significant at the 99% level after both 5 hours and 24 hours. These results clearly indicate that the present invention can deliver a highly effective deodorancy benefit.

In an analogous test, 0.1% w/w AEVG in aqueous ethanol (20:80 w/w) was tested against a control product (20:80 w/w). On odour assessment after 24 hours, reduced odour was found from use of the AEVG product at the 99% level on both male and female panellists.

In a further test, a composition containing 0.25% AEVG, 1% fragrance, 80% ethanol and water was tested against a control product lacking the AEVG. On odour assessment of male panellists after 24 hours, reduced odour was found from use of the AEVG product at the 99% level, illustrating the benefit for compositions comprising both AEVG and fragrance over a simple fragranced ethanolic deodorant.

In a further test, a composition containing 0.25% AEVG, 0.29% Cosmocil CQ (PHMB chloride ex Arch Chemicals Inc.), 80% ethanol and water was tested against a control product lacking the AEVG. On odour assessment of female panellists after 24 hours, reduced odour was found from use of the AEVG product at the 99% level, illustrating the benefit for compositions comprising both AEVG and an additional organic anti-microbial.

In a further test, a composition containing 0.25% AEVG, 20% Aloxicoll L (aluminium chlorohydrate, ex BK Giulini GmbH), and water was tested against a control product lacking the AEVG. On odour assessment of female panellists after 24 hours, reduced odour was found from use of the AEVG product at the 95% level, illustrating the benefit for compositions comprising both AEVG and an additional inorganic anti-microbial.

The compositions indicated in the following tables are examples according to the invention and may be prepared by methods known in the art.

**Table 2: Squeeze Spray Compositions**

| **Component** | **Example 1** | **Example 2** |
|---|---|---|
| | | |
| Ethanol | 60 | 70 |
| AEVG | 0.2 | - |
| RhB | - | 0.25 |
| Fragrance | 1.2 | 1.3 |
| Glycerol | 1.0 | 1.0 |
| Water | To 100 | To 100 |

**Table 3: Roll-on Compositions**

| **Component** | **Example 3** | **Example 4** |
|---|---|---|
| | | |
| Ethanol | 55 | 65 |
| AEVG | 0.1 | 0.2 |
| DTPA | - | 1.0 |
| Sodium hydroxide | - | 0.34 |
| Fragrance | 1.4 | 1.4 |
| Klucel M | 0.65 | 0.65 |
| Water | To 100 | To 100 |

**Table 4: Solid Compositions**

| Component | Example 5 (Soft Solid) | Example 6 (Gel Stick) |
|---|---|---|
| AEVG | 0.3 | 0.15 |
| RhB | - | 0.15 |
| Perfume | 1.0 | 1.2 |
| Dextrin Palmitate | 10 | - |
| Finsolv TN¹ | To 100 | - |
| Propylene Glycol | - | 22.5 |
| Dipropylene Glycol | - | 40.0 |
| Sodium Stearate | - | 5.5 |
| Tetronic 1307² | - | 3.0 |
| Water | - | To 100 |

| | | |
|---|---|---|
| 1. C12-C15 alkyl benzoate, ex Finetex. 2. Poloxamine 1307, ex BASF. | | |

**Table 5: Aerosol Compositions**

| **Component** | **Example** | |
|---|---|---|
| | **7** | **8** |
| AEVG | 0.2 | 0.25 |
| DC 245 | 11.9 | - |
| Bentone 38V | 0.6 | - |
| Propylene carbonate | 0.2 | - |
| Water | - | 30 |
| Ethanol | - | 20 |
| Dimethyl ether | - | To 100 |
| CAP40 | To 100 | - |

## Claims

1. A non-therapeutic method of achieving a deodorancy benefit comprising the application to the surface of the human body of a compound of formula I_{:} R¹R²N-X-O-CH=CH-CH(NH₂)CO₂H or formula II: R¹R²N-X-O-CH₂CH₂-CH(NH₂)CO₂H, where X is an optionally substituted alkylene group comprising two carbons including the possibility that the alkylene group has 3, 4, 5 or more carbon atoms arranged in linear fashion and R¹ and R² are independently H or CH₃.

2. A non-therapeutic method of achieving a deodorancy benefit comprising the application to the surface of the human body of a compound which is an ester, amide or salt of a compound of formula I or II that forms a compound of formula I or II, as described in claim 1, upon the surface of the human body.

## Patentansprüche

1. Nicht-therapeutisches Verfahren zum Erzielen eines Desodorierungseffektes, bei dem auf die Oberfläche des menschlichen Körpers eine Verbindung mit der Formel I: R¹R²N-X-O-CH=CH-CH(NH₂)CO₂H oder der Formel II:
R¹R²N-X-O-CH₂CH₂-CH(NH₂)CO₂H
aufgebracht wird, wobei X eine gegebenenfalls substituierte Alkylengruppe mit 2 Kohlenstoffatomen ist, wobei die Möglichkeit eingeschlossen ist, dass die Alkylengruppe 3, 4, 5 oder mehr Kohlenstoffatome aufweist, die linear angeordnet sind, und R¹ und R² unabhängig voneinander H oder CH₃ sind.

2. Nicht-therapeutisches Verfahren zum Erzielen eins Desodorierungseffektes, bei dem auf die Oberfläche des menschlichen Körpers eine Verbindung aufgebracht wird, die ein Ester, Amid oder Salz einer Verbindung der Formel I oder II ist, der bzw. das auf der Oberfläche des menschlichen Körpers eine Verbindung der Formel I oder II gemäß Anspruch 1 bildet.

## Revendications

1. Procédé non thérapeutique d'obtention d'un bénéfice de caractère déodorant comprenant l'application sur la surface du corps humain d'un composé de formule I : R¹R²N-X-O-CH=CH-CH(NH₂)CO₂H ou de formule II : R¹R²N-X-O-CH₂CH₂-CH(NH₂)CO₂H, où X représente un groupe alkylène éventuellement substitué comprenant deux carbones incluant la possibilité que le groupe alkylène comporte 3, 4, 5 atomes ou plus de carbone arrangés de façon linéaire et R¹ et R² représentent indépendamment H ou CH₃.

2. Procédé non thérapeutique d'obtention d'un bénéfice de caractère déodorant comprenant l'application sur la surface du corps humain d'un composé qui est un ester, un amide ou un sel d'un composé de formule 1 ou II qui forme un composé de formule I ou II, tel que décrit dans la revendication 1, sur la surface du corps humain.
